(19) Europäisches Patentamt
European Patent Office
Office européen des brevets

(11) **EP 3 349 030 A1**

(12) **EUROPEAN PATENT APPLICATION**

(43) Date of publication:
**18.07.2018 Bulletin 2018/29**

(51) Int Cl.:
***G01R 33/28*** *(2006.01)*

(21) Application number: **17204473.7**

(22) Date of filing: **29.11.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.12.2016 KR 20160166343**

(71) Applicant: **Samsung Electronics Co., Ltd.**
**Suwon-si, Gyeonggi-do (KR)**

(72) Inventor: **Verghese, George**
**Yongin-si, Gyeonggi-do (KR)**

(74) Representative: **Gulde & Partner**
**Patent- und Rechtsanwaltskanzlei mbB**
**Wallstraße 58/59**
**10179 Berlin (DE)**

(54) **MAGNETIC RESONANCE IMAGING APPARATUS AND CONTROLLING METHOD THEREFOR**

(57) A magnetic resonance imaging (MRI) apparatus may be provided. The MRI apparatus may include: a magnet assembly configured to generate a magnetic field in an inner space of the magnet assembly; a radiofrequency (RF) reception coil configured to receive a magnetic resonance (MR) signal excited from an object to which the magnetic field is applied; a magnetic sensor disposed at the RF reception coil and configured to detect a voltage value indicating a static field generated by the magnet assembly; and a processor configured to determine whether the RF reception coil is placed in a normal position based on the voltage value transmitted from the magnetic sensor when the RF reception coil enters the inner space of the magnetic assembly.

**FIG. 8**

## Description

## BACKGROUND

### 1. Field

Apparatuses and methods consistent with exemplary embodiments relate to a magnetic resonance imaging apparatus capable of generating a magnetic resonance image of an object and a controlling method thereof.

### 2. Description of the Related Art

In general, medical imaging apparatuses are configured to acquire information on a patient and provide an image. Medical imaging apparatuses include X-ray apparatuses, ultrasonic diagnostic apparatuses, computed tomography apparatuses, and magnetic resonance imaging (MRI) apparatuses.

Among these, magnetic resonance imaging apparatuses may play an important role in fields using medical images because they have relatively less restrictive image capturing conditions and provide sufficient contrast in soft tissues and various diagnostic information images.

Magnetic Resonance Imaging (MRI) images the density and the physicochemical properties of atomic nuclei by generating nuclear magnetic resonance of hydrogen atomic nuclei in the body using magnetic fields which are not harmful to human bodies and Radio Frequencies (RF) which are non-ionizing radiation.

In detail, the MRI apparatus images the inside of an object by converting energy discharged from atomic nuclei into a signal by supplying a designated frequency and energy to the atomic nuclei in a state in which a designated magnetic field is applied to the inside of a bore.

In particular, a radio frequency (RF) coil may be used to receive energy emitted from the atomic nuclei, and the RF coil may be separated from a patient table. The RF coil is usually kept separated from the patient table, and the RF coil may be connected to the patient table when a magnetic resonance image is taken.

Meanwhile, when the RF coil is moved or the direction of the mounted RF coil is dislocated during the magnetic resonance imaging, it may be difficult to obtain a desired magnetic resonance image. In addition, when the desired magnetic resonance image is not obtained, the magnetic resonance imaging may have to be performed again and thus the examination time and the patient's inconvenience may increase.

## SUMMARY

Exemplary embodiments address at least the above problems and/or disadvantages and other disadvantages not described above. Also, the exemplary embodiments are not required to overcome the disadvantages described above, and may not overcome any of the problems described above.

One or more exemplary embodiments provide a magnetic resonance imaging apparatus capable of preventing a re-imaging, which occurs when a RF coil is not properly mounted, by determining a position or an orientation of the RF coil in a bore based on a detection value of a magnetic sensor contained in the RF coil, and capable of securing the patient safety by preventing the over-heated RF coil, and a controlling method thereof.

In accordance with one aspect of the present disclosure, A magnetic resonance imaging (MRI) apparatus include a magnet assembly generating a magnetic field in an inner space thereof; a radiofrequency (RF) reception coil receiving an magnetic resonance (MR) signal excited from an object to which a magnetic field generated by the magnet assembly is applied; a magnetic sensor provided in the RF reception coil and configured to detect a voltage value based on a static field generated by the magnet assembly; and a controller determining whether a direction of the RF reception coil entering inside the magnet assembly, is normal based on the voltage value transmitted from the magnetic sensor.

The controller may determine that the direction of the RF reception coil is normal when the voltage value transmitted from the magnetic sensor exceeds a predetermined reference voltage value.

The RF reception coil may be separated from the magnet assembly and closely attached to the object.

The RF reception coil may include a DC conductor provided together with the magnetic sensor.

A transfer table may transfer the object to the inner space of the magnet assembly.

The controller may stop the movement of the transfer table when determining that the direction of the RF reception coil is abnormal.

The MRI apparatus may further include an image processor generating an image signal by receiving data related to MR signals transmitted by the RF reception signal; and a display displaying an MR image generated by the image processor, wherein when determining that the direction of the RF reception coil is abnormal, the controller allows the display to display a warning image.

The MRI apparatus may further include a sound output portion warning a user, wherein the sound output portion outputs a warning sound to the user when it is determined that the direction of the RF reception coil is abnormal.

The magnetic sensor may include a first magnetic sensor provided in the RF reception coil and a second magnetic sensor provided in the RF reception coil to be apart from the first magnetic sensor.

The controller may determine the direction of the RF reception coil by comparing a first detection value of the first magnetic sensor and a second detection value of the second magnetic sensor with the predetermined reference voltage value.

The controller may determine a position of the RF reception coil by comparing detection values of the first magnetic sensor and the second magnetic sensor.

In accordance with another aspect of the present disclosure, a controlling method of a magnetic resonance imaging (MRI) apparatus an operation in which a magnet assembly generates a magnetic field therein; an operation in which a radiofrequency (RF) reception coil receives an magnetic resonance (MR) signal excited from an object to which a magnetic field is applied; an operation in which a magnetic sensor detects a voltage value based on a static field ; and an operation of determining whether a direction of the RF reception coil entering inside the magnet assembly, is normal based on the voltage value transmitted from the magnetic sensor.

The determination may include determining that the direction of the RF reception coil is normal when the voltage value transmitted from the magnetic sensor exceeds a predetermined reference voltage value.

The RF reception coil may be separated from the magnet assembly and closely attached to the object.

The RF reception coil may include a DC conductor provided together with the magnetic sensor.

The control method may further include stopping the movement of the object into the inner space of the magnet assembly when it is determined that the direction of the RF reception coil is abnormal.

The magnetic sensor may include a first magnetic sensor provided in the RF reception coil and a second magnetic sensor provided in the RF reception coil to be apart from the first magnetic sensor.

The determination may include determining the direction of the RF reception coil by comparing a first detection value of the first magnetic sensor and a second detection value of the second magnetic sensor with the predetermined reference voltage value.

The determination may include determining a position of the direction of the RF reception coil by comparing detection values of the first magnetic sensor and the second magnetic sensor.

The control method may further include outputting a warning sound or a warning image to the user when it is determined that the direction of the RF reception coil is abnormal.

According to an aspect of an exemplary embodiment, there is provided a magnetic resonance imaging (MRI) apparatus including: a magnet assembly configured to generate a magnetic field in an inner space of the magnet assembly; a radiofrequency (RF) reception coil configured to receive a magnetic resonance (MR) signal excited from an object to which the magnetic field is applied; a magnetic sensor disposed at the RF reception coil and configured to detect a voltage value indicating a static field generated by the magnet assembly; and a processor configured to determine whether the RF reception coil is placed in a normal position based on the voltage value transmitted from the magnetic sensor when the RF reception coil enters the inner space of the magnetic assembly.

The processor may be further configured to determine that the RF reception coil is placed in the normal position in response to the voltage value transmitted from the magnetic sensor exceeding a predetermined reference voltage value.

The RF reception coil may be separated from the magnet assembly and disposed closer to the object than the magnet assembly.

The RF reception coil may include a direct current (DC) conductor that is coupled to the magnetic sensor.

The MRI apparatus may further include a transfer table configured to transfer the object to the inner space of the magnet assembly.

The processor may be further configured to stop the transfer table from moving in response to determining that the RF reception coil is placed in an abnormal position.

The MRI apparatus may further include: an image processor configured to generate an MR image based on MR signals transmitted from the RF reception coil; and a display configured to display the MR image generated by the image processor, wherein the processor is further configured to control the display to display a warning image in response to determining that the RF reception coil is placed in an abnormal position.

The MRI apparatus may further include: a sound output interface configured to output a warning sound to a user in response to determining that the RF reception coil is placed in an abnormal position.

The magnetic sensor may include a first magnetic sensor and a second magnetic sensor that are disposed at the RF reception coil, and the first magnetic sensor may be disposed apart from the second magnetic sensor.

The processor may be further configured to determine whether the RF reception is placed in the normal position by comparing a first detection value of the first magnetic sensor and a second detection value of the second magnetic sensor with a predetermined reference voltage value.

The processor may be further configured to determine whether the RF reception is placed in the normal position by comparing a first detection value of the first magnetic sensor with a second detection value of the second magnetic sensor.

According to an aspect of an exemplary embodiment, there is provided a control method of a magnetic resonance imaging (MRI) apparatus, including: generating, by a magnet assembly, a magnetic field in an inner space of the magnetic assembly; receiving, by a radiofrequency (RF) reception coil, a magnetic resonance (MR) signal excited from an object to which the magnetic field is applied; detecting, by a magnetic sensor, a voltage value indicating a static field generated by the magnetic assembly; and determining whether the RF reception coil is placed in a normal position based on the voltage value transmitted from the magnetic sensor when the RF reception coil enters the inner space of the magnetic assembly.

The determining may include determining that the RF reception coil is placed in the normal position in response to the voltage value transmitted from the magnetic sensor exceeding a predetermined reference voltage value.

The control method may further include: stopping the object from moving into the inner space of the magnet assembly in response to determining that the RF reception coil is placed in an abnormal position.

The magnetic sensor may include a first magnetic sensor and a second magnetic sensor that are disposed at the RF reception coil, and the first magnetic sensor may be disposed apart from the second magnetic sensor, and the determining may include determining whether the RF reception is placed in the normal position by comparing a first detection value of the first magnetic sensor and a second detection value of the second magnetic sensor with a predetermined reference voltage value.

The magnetic sensor may include a first magnetic sensor and a second magnetic sensor that are disposed at the RF reception coil, and the first magnetic sensor may be disposed apart from the second magnetic sensor, and the determining may include determining whether the RF reception is placed in the normal position by comparing a first detection value of the first magnetic sensor with a second detection value of the second magnetic sensor.

The control method may further include: outputting a warning sound or a warning image to the user in response to determining that the RF reception coil is placed in an abnormal position.

According to an aspect of an exemplary embodiment, there is provided a magnetic resonance imaging (MRI) apparatus including: a magnet assembly configured to generate a magnetic field in a bore; a radiofrequency (RF) reception coil that is disposed closer to an object than the magnetic assembly; a magnetic sensor disposed at the RF reception coil and configured to detect the magnetic field generated by the magnet assembly; and a processor configured to determine whether an increase rate of a strength of the detected magnetic field is less than a threshold increase rate while the object is moving into the bore, and indicate that the RF reception coil is in an abnormal position in response to the increase rate of the strength of the detected magnetic field being less than the threshold increase rate.

The magnetic sensor may include a plurality of sub-magnetic sensors that are disposed apart from each other, and the processor may be further configured to determine whether a difference between magnetic field directions of the plurality of sub-magnetic sensors is within a predetermined direction difference range, and indicate that the RF reception coil is in the abnormal position in response to the difference between the magnetic field directions is outside the predetermined direction difference range.

The magnetic sensor may include a plurality of sub-magnetic sensors that are disposed apart from each other, and the processor may be further configured to determine whether a difference between magnetic field strengths of the plurality of sub-magnetic sensors is within a predetermined strength difference range, and indicate that the RF reception coil is in the abnormal position in response to the difference between the magnetic field strengths is outside the predetermined strength difference range.

BRIEF DESCRIPTION OF THE DRAWINGS

The above and/or other aspects will be more apparent by describing certain exemplary embodiments, with reference to the accompanying drawings, in which:

FIG. 1A is a view schematically illustrating a magnetic resonance imaging (MRI) system.
FIG. 1B is a control block diagram illustrating the MRI system in accordance with an exemplary embodiment.
FIG. 2 illustrates an MRI scanner cutaway.
FIG. 3 is a view illustrating a cross-section of an examination region, in which an object is placed, along the X, Y and Z axes.
FIG. 4 is a view illustrating the structure of a magnet assembly and a gradient coil portion.
FIG. 5 is a view illustrating a pulse sequence regarding operations of respective gradient coils of the gradient coil portion.
FIG. 6 is a view illustrating a radio frequency (RF) reception coil and an overheating problem according to an exemplary embodiment.
FIG. 7 is a view illustrating a hall effect sensor according to an exemplary embodiment.
FIG. 8 is a view illustrating a RF reception coil according to an exemplary embodiment.
FIGS. 9A and 9B illustrate an operation of detecting the direction of a breast coil in a static field.
FIG. 10A, 10B, and 10C illustrate a RF reception coil according to another exemplary embodiment.
FIG. 11 is a view illustrating a RF reception coil and a transfer table in accordance with an exemplary embodiment.
FIG. 12 is a flow chart illustrating an operation of the MRI apparatus.
FIG. 13 is a flowchart illustrating a method for determining the orientation of the RF reception coil.
FIG. 14 is a flowchart illustrating a method for determining the position of the RF reception coil.

DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

Terms such as "part" and "portion" may be embodied as hardware or software. According to exemplary embodiments, a plurality of "part" and "portion" may be implemented as a single component or a single "part" and "portion" may include a plurality of components.

In the description, an "image" may include a medical image acquired by medical imaging apparatuses, for example, magnetic resonance imaging apparatuses (MRI), computed tomography apparatuses (CT), ultrasonic diagnostic apparatuses or X-ray apparatuses.

The term "object" may represent an object of imaging and include human, animals or a part thereof. For example, an object may include a part of body (organ) or phantom.

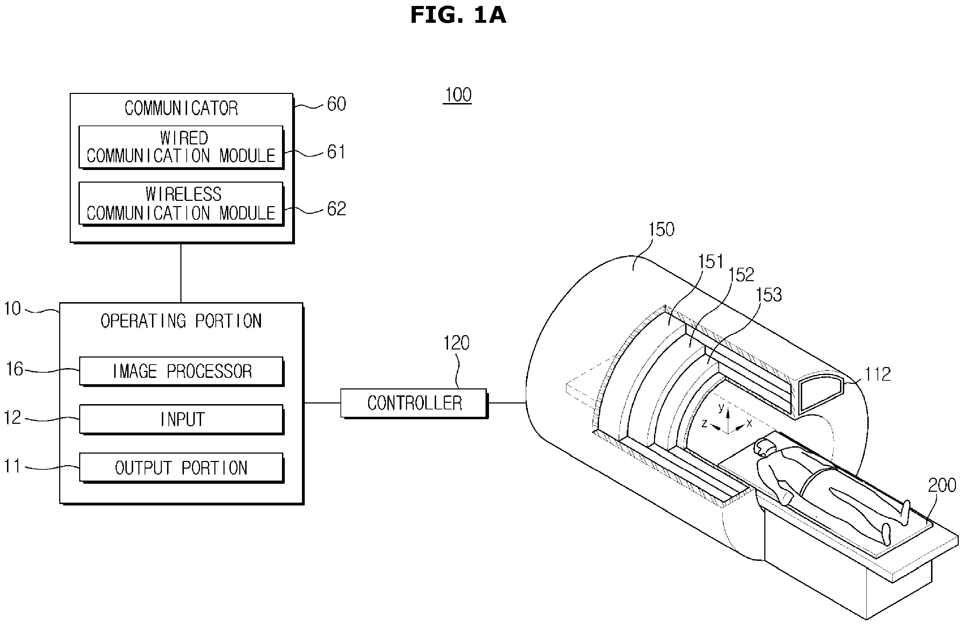

FIG. 1A is a view schematically illustrating an magnetic resonance imaging (MRI) system. Referring to FIG. 1A, an MRI system 100 may include a controller 120 and a scanner 150. The controller 120 may be also referred to as a processor. The controller 120 may be independently implemented as illustrated in FIG. 1A. Alternatively, the controller 120 may be implemented with a plurality of physically separated components that are disposed at different positions in the MRI system 100. Hereinafter each component of the MRI system 100 will be described in detail.

The scanner 150 may be formed in a shape that provides a void space inside the scanner 150, and thus an object allows to be inserted thereinto (e.g., a bore or tunnel shape). Inside the scanner 150, a static field and a gradient field are formed and an RF signal is emitted.

The scanner 150 may include a static field coil portion 151, a gradient coil portion 152, a RF transmission coil 153, a transfer table 200 and a display 112.

The static field coil portion 151 forms a static magnetic field for aligning the direction of the magnetic dipole moments of atomic nuclei in an object. The static field coil portion 151 may be implemented as a permanent magnet or a superconducting magnet using a cooling coil.

The gradient coil portion 152 is connected to the controller 120. The gradient coil portion 152 forms a gradient magnetic field by applying a gradient field to the static magnetic field according to a control signal transmitted from the controller 120. The gradient coil portion 152 includes X, Y, and Z coils that form mutually orthogonal X, Y, and Z-axis gradient magnetic fields. The gradient coil portion 152 generates a gradient signal corresponding to an imaging position so as to differently induce resonance frequencies according to a part of the object.

The RF transmission coil 153 is connected to the controller 120 to radiate an RF signal to an object according to the control signal transmitted from the controller 120. The RF transmission coil 153 may receive an magnetic resonance (MR) signal emitted from the object. The RF transmission coil 153 may transmit an RF signal having a frequency equal to the frequency of the precession, to the target atomic nuclei in the precession, and then stop the transmission of the RF signal and receive an MR signal emitted from the object.

The RF transmission coil 153 generates an electromagnetic wave having a radio frequency corresponding to the type of the atomic nuclei, and an RF reception coil 170 receives electromagnetic waves radiated from the atomic nuclei.

According to an exemplary embodiment, the RF reception coil 170 may be mounted to the object. For example, an additional coil such as a head coil, a spine coil, a torso coil, and a knee coil may be used according to an imaging part and a mounting part. A detailed description related thereto will be described later with reference to other drawings.

The display 112 may be provided inside and/or outside of the scanner 150. The display 112 may be controlled by the controller 120 and the display 112 may provide information related to the medical imaging to the user or the object.

In addition, the scanner 150 may include an object monitoring information acquisition portion configured to acquire and transmit monitoring information related to an object status. For example, the object monitoring information acquisition portion may acquire monitoring information related to the object from a camera for imaging the movement and position of the object, a breathing meter for measuring the respiration of the object, an electrocardiogram (ECG) measuring instrument for measuring the electrocardiogram of the object, or a body temperature measuring instrument for measuring a temperature of the object. The object monitoring information acquisition portion may transmit the monitoring information to the controller 120. Accordingly, the controller 120 may control the operation of the scanner 150 using the monitoring information about the object. Hereinafter, the controller 120 will be described.

The controller 120 may control the overall operation of the scanner 150.

The controller 120 may control the sequence of signals formed within the scanner 150. The controller 120 may control the gradient coil portion 152 and the RF transmission coil 153 and the RF reception coil 170 according to the pulse sequence received from the operating portion 10 or a designed pulse sequence.

Pulse sequence includes all kinds of information for controlling the gradient coil portion 152, the RF transmission coil 153, and the RF reception coil 170 (e.g., an intensity of a pulse signal applied to the gradient coil portion 152, an application duration, and an application timing).

The controller 120 may control the gradient magnetic field formation of the gradient coil portion 152 by controlling a waveform generator generating a gradient waveform (i.e., a current pulse) according to a pulse sequence, and a gradient amplifier amplifying the generated current pulse and transmitting the amplified current pulse to the gradient coil portion 152.

The controller 120 may control the operation of the RF transmission coil 153 and the RF reception coil 170. For example, the controller 120 may supply an RF pulse having a resonance frequency to the RF transmission coil 153 so as to irradiate the RF signal and the controller 120 may receive the MR signal through the RF reception coil 170. In particular, the controller 120 may control an operation of a switch (e.g., a transmission/reception switch), which is configured to change the transmission and reception direction of the RF signal and the MR signal, by a control signal so as to control the irradiation of the RF signal and the reception of the MR signal according to an operation mode.

The controller 120 may control the movement of the transfer table 200 on which the object lies down. Before the imaging is performed, the controller 120 may move the transfer table 200 in advance in accordance with a target part of the object.

The controller 120 may control the display 112. For example, the controller 120 may control ON/OFF state of the display 112 or a screen displayed on the display 112.

The controller 120 may be implemented using a memory storing an algorithm for controlling an operation of components in the MRI system 100 and data related to programs implementing the algorithm, and a processor performing the above mentioned operation using the data stored in the memory. The memory and the processor may be implemented in separate chips, or a single chip.

A detailed description related to the controller 120 will be described later in detail with the drawings.

The operating portion 10 may control the overall operation of the MRI system 100. The operating portion 10 may include an image processor 16, an input 12, and an output portion 11.

The image processor 16 may store the MR signal received from the controller 120 by using the memory, and generate an image data about the object, from the stored MR signal by applying the image reconstruction technique by using the image processor.

For example, when a k- space data is completed by filling digital data in a k- space of the memory (referred to as a Fourier space or a frequency space), the image processor 16 may restore the k-space data to image data by applying various image restoration techniques (e.g., by performing inverse Fourier transform on the k-space data) by using the image processor.

Further, various signal processing which is applied to the MR signal by the image processor 16, may be performed in parallel. For example, the image processor 16 may restore an image data by processing a plurality of MR signals received by a multi-channel RF coil, in parallel. Further, the image processor 16 may store the restored image data in the memory or the controller 120 may store the restored image data in an external server through a communicator 60, as described later.

The input 12 may receive a control command about the overall operation of the MRI system 100 from a user. For example, the input 12 may receive object information, parameter information, scan conditions, and information on pulse sequences from a user. The input 12 may be implemented as a keyboard, a mouse, a trackball, a voice recognition unit, a gesture recognition unit, or a touch screen.

The output portion 11 may output the image data generated by the image processor 16. The output portion 11 may output a user interface (UI) configured to allow a user to input a control command related to the MRI system 100. The output portion 11 will be described later with reference to the drawings.

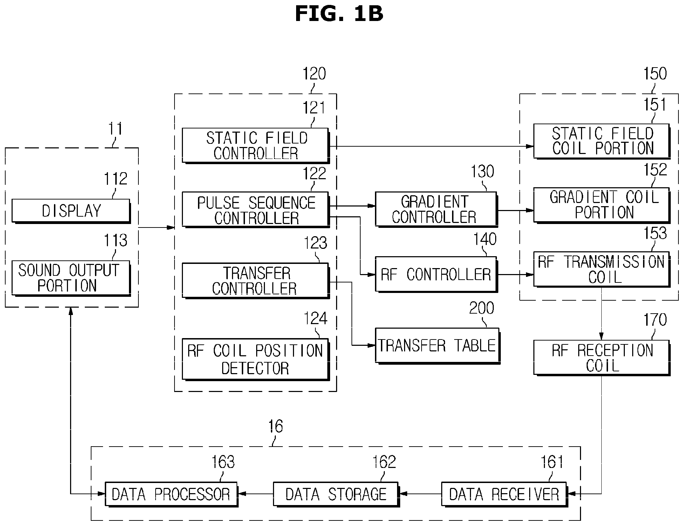

FIG. 1A illustrates that the operating portion 10 and the controller 120 are separated from each other, but the operating portion 10 and the controller 120 may be contained in a single apparatus, as mentioned above. Also, the processes performed by each of the operating portion 10 and the controller 120 may be performed by different components. For example, the image processor 16 may convert an MR signal received by the controller 120 into a digital signal or the controller 120 may convert an MR signal into a digital signal by itself. FIG. 1B is a control block diagram illustrating the MRI system in accordance with an exemplary embodiment. Hereinafter the operation of the MRI system (hereinafter referred to as a magnetic resonance imaging (MRI) apparatus) will be described in detail with reference to FIG. 1B. Particularly, it is assumed that the RF reception coil is separated from the magnet assembly.

Referring to FIG. 1B, according to an exemplary embodiment, the MRI apparatus includes the scanner (hereinafter referred to as a magnet assembly 150) forming a magnetic field and generating a resonance phenomenon on atomic nuclei, the controller 120 controlling the operation of the magnet assembly 150 and determining a position and an orientation of the RF reception coil 170, the image processor 16 generating an MR image based on an echo signal (i.e., an MR signal) generated from atomic nuclei, and the RF reception coil 170 receiving the MR signal generated by the magnet assembly and transmitting the MR signal to the image processor 16. The magnet assembly 150 includes the static field coil portion 151 forming a static field in the inner space of the magnet assembly 150, the gradient coil portion 152 forming a gradient magnetic field by generating a gradient field to the static field, and the RF transmission coil 153 applying the RF pulse. When an object Ob is placed in the inner space of the magnet assembly 150, the static field, the gradient field and the RF pulse may be applied to the object. The atomic nuclei constituting the object Ob is excited by the applied RF pulse, and an echo signal is generated therefrom.

The RF reception coil 170 may receive an RF signal (i.e., an MR signal) emitted from the excited atomic nuclei. The RF reception coil 170 may act as an antenna to receive an RF signal coming out of the object Ob and to transmit corresponding data to the image processor 16 that generates MRI images. Particularly, the RF reception coil 170 may transmit an RF signal having the same frequency as the frequency of the precession, toward the target atomic nuclei in the precession, to the object Ob and then stop the transmission of the RF signal and receive an MR signal emitted from the object Ob.

In general, the RF reception coil 170 may be fixed to or removably attached to the bore. The removable RF reception coil 170 may include an RF coil for a portion of the object Ob, including a head RF coil, a chest RF coil, a leg RF coil, a neck RF coil, a shoulder RF coil, a wrist RF coil, and an ankle RF coil.

An example of the RF reception coil 170, which is removably attached to the magnet assembly 150, is a surface coil configured to receive an MR signal excited in the some portion of the object Ob. The surface coil may have a relatively smaller size than the volume coil, and have a two-dimensional surface. Therefore, the surface coil may have a significantly higher signal to noise ratio about adjacent parts.

Another example of the RF reception coil 170 is an array coil in which a plurality of surface coils is arranged in one-dimension or two-dimensions to extend a reception region. The array coil may have various array shapes according to a target part, and the array coil may be classified into a head type, a head and neck type, a chest type, a spine type, an abdomen type and a leg type. Since the relative positions of the surface coils forming the array coil are different, the phases of the signals received by the surface coils also differ. Therefore, when an image is reconstructed by combining signals received by each surface coil, it may be possible to obtain an image having a high signal-to-noise ratio by considering the receive phase of the surface coil.

According to an exemplary embodiment, the RF reception coil 170 may include a magnetic sensor. Particularly, the magnetic sensor represents a sensor configured to detect a magnetic field applied by the magnet assembly 150.

Particularly, the magnetic sensor according to an exemplary embodiment may include a hall effect sensor 171 configured to detect the magnetic field based on the hall-effect. The hall effect sensor 171 may be attached to the RF reception coil 170 or may be in a built-in form. Hereinafter, for convenience of explanation, the magnetic sensor mounted on the RF receiving coil will be referred to as the hall effect sensor 171.

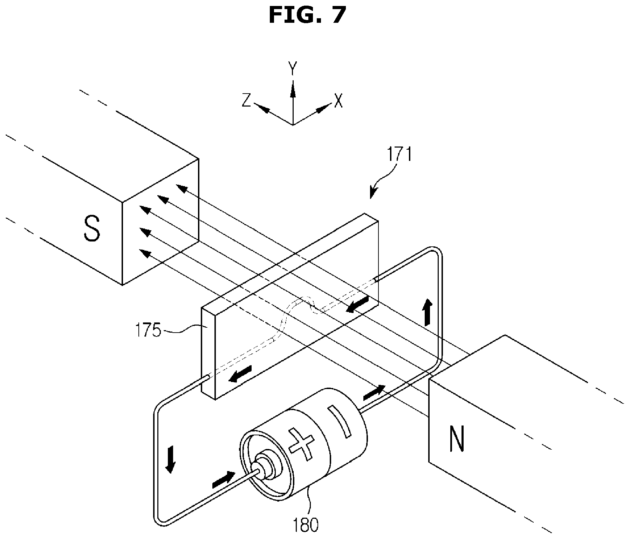

When the RF reception coil 170 wraps around the body part of the object to be scanned, the hall effect sensor 171 detects a hall voltage value due to a magnetic field formed inside the bore, and then the hall effect sensor 171 transmits the hall voltage value to the controller 120. The hall effect sensor 171 will be described later in detail with reference to FIG. 7.

The controller 120 includes a static field controller 121 controlling the intensity and direction of the static field formed by the static field coil portion 151, a pulse sequence controller 122 controlling the gradient coil portion 152 and the RF transmission coil 153 by designing the pulse sequence, a transfer controller 123 controlling the transfer table 200 configured to move the object Ob to the cavity, and a RF coil position detector 124 detecting whether the RF reception coil 170 is mounted or not, or the orientation of the RF reception coil 170.

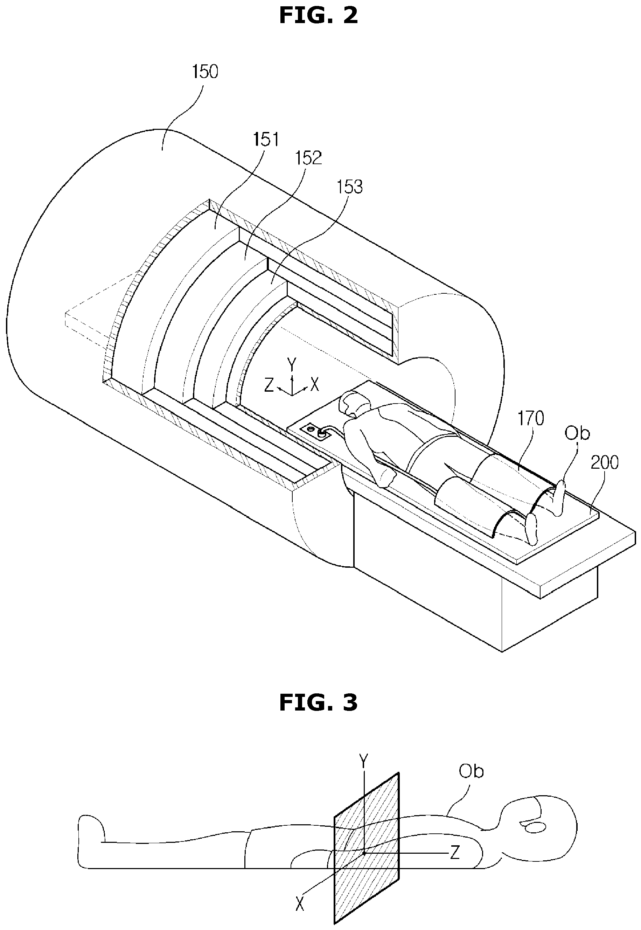

A detailed description of the operation of the static field controller 121 and the pulse sequence controller 122 will be described later with reference to FIG. 2.

The transfer controller 123 controls the transfer table 200 on which the object Ob lies, according to an input signal that is input via an operation console 111 by a user. In addition, when the RF reception coil 170 is not properly mounted, the transfer controller 123 may stop the transfer table 200 from moving by interrupting an input received from the operation console 111.

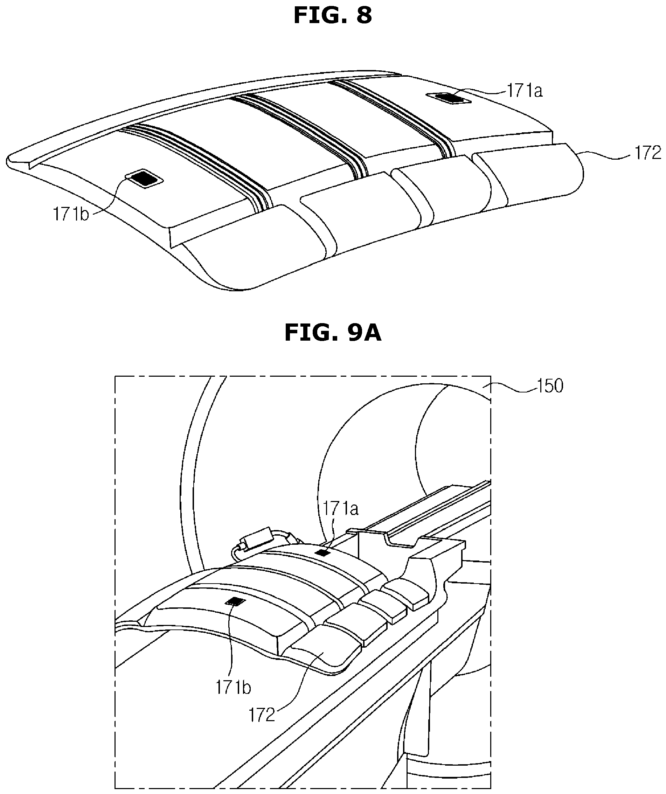

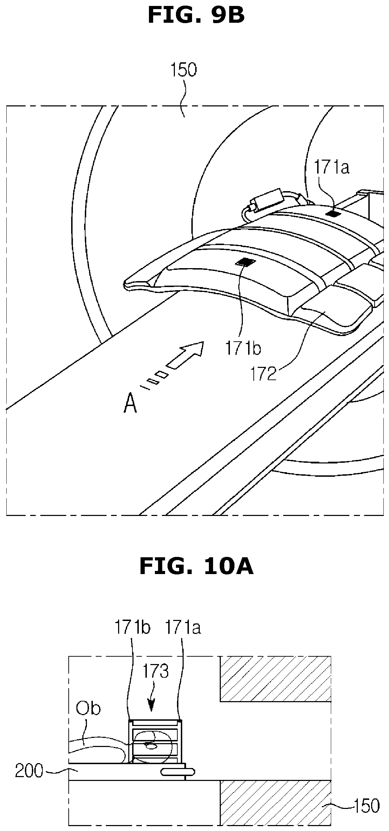

The RF coil position detector 124 receives a detection value from the hall effect sensor 171 contained in the RF reception coil 170, and determines whether the RF reception coil 170 is properly mounted to the object Ob. Particularly, the RF coil position detector 124 may determine the orientation of the RF reception coil using the detection value of the hall effect sensor 171, and also determine the position of the RF reception coil based on the detection value. The determination process of the RF coil position detector 124 will be described later with reference to FIGS. 9A and 9B.

As mentioned above, the controller 120 is a processor configured to control the overall operation of the MRI apparatus 100. The controller 120 may be realized as a plurality of hardware chips that respectively correspond to the static field controller 121, the pulse sequence controller 122, the transfer controller 123 and the RF coil position detector 124, but is not limited thereto. The static field controller 121, the pulse sequence controller 122, the transfer controller 123 and the RF coil position detector 124 of the controller 120 may represent software modules and may be integrated into a single system on chip (SOC) in a hardware manner.

The controller 120 may be implemented using a memory storing an algorithm for controlling an operation of components in the MRI apparatus 100 and data related to programs implementing the algorithm, and a processor performing the above mentioned operation using the data stored in the memory. The memory and the processor may be implemented in separate chips, or a single chip.

The MRI apparatus 100 may include a gradient controller 130 applying a gradient signal to the gradient coil portion 152 and an RF transmitter 140 applying an RF signal to the RF transmission coil 153. According to the control of the pulse sequence controller 122, the gradient controller 130 and the RF transmitter 140 may adjust the gradient fields formed inside the magnet assembly 150 and the RF applied to the atomic nuclei.

The image processor 16 includes a data receiver 161 collecting data regarding a spin echo signal, i.e., an MR signal generated from the atomic nuclei, a data storage 162 storing data received by the data receiver 161, and a data processor 163 generating an MR image by processing the data stored in the data storage 162.

The data receiver 161 may include a pre-amplifier amplifying the MR signal received by the RF reception coil 170, a phase detector receiving the MR signal transmitted from the pre-amplifier and then detecting a phase, and an analog/digital (A/D) converter converting an analog signal acquired through phase detection into a digital signal. Further, the data receiver 161 transmits the MR signal converted into the digital signal to the data storage 162.

A data space constructing a two-dimensional (2D) Fourier space is formed in the data storage 162, and when the entire data generated by completing scanning is completely stored, the data processor 163 reconfigures the image of the object Ob by performing 2D inverse Fourier transform on data in the 2D Fourier space. The reconfigured image is displayed on the display 112.

Further, the MRI apparatus 100 may include a user operation portion 110, and may thus receive control instructions regarding the overall operation of the MRI apparatus 100 from a user and, particularly, receive instructions regarding a scan sequence from the user and generate a pulse sequence.

The user operation portion 110 may include the operation console 111 that allows a user to operate a system, the display 112 displaying a control state and displaying the image generated by the image processor 16 to allow the user to diagnose the health state of the object, and a sound output portion (e.g., a sound output interface) 113 warning a fact that the RF reception coil 170 is not properly mounted , to a user.

The display 112 may display an output screen associated with the operation of the MRI apparatus 100 as well as an image generated by the image processor 16. Particularly, when the RF reception coil 170 is not properly mounted to the object Ob, or when the RF reception coil 170 is placed without the orientation for receiving the static field, it may possible to display a warning image on the display screen to warn the user.

The display 112 may be implemented by a cathode ray tube (CRT), a digital light processing (DLP) panel, a plasma display penal (PDP), a liquid crystal display (LCD) panel, an electro luminescence (EL) panel, an electrophoretic display (EPD) panel, an electrochromic display (ECD) panel, a light emitting diode (LED) panel or an organic light emitting diode (OLED) Panel, but is not limited thereto.

As mentioned above, the display 112 may serve as the input 12 when the display 112 includes a touch screen panel (TSP).

The sound output portion 113 is a module operating a component of the output portion 11 related to the speaker outputting a warning sound among the output operation performed by the MRI apparatus 100. That is, the sound output portion 113 may output a sound, a warning sound and voice guidance to the user.

Particularly, when the RF reception coil 170 is not properly mounted on the object Ob, or when the RF reception coil 170 is placed without the orientation for receiving the static field, the MRI apparatus 100 may warn the user through the sound output portion 113.

In addition, the MRI apparatus 100 may include a component that is not described herein. For example, a magnetic resonance (MRI) apparatus 100 may include a hybrid RF coil capable of simultaneously performing RF transmission and reception. In this case, a hall effect sensor 171 may be provided in the hybrid RF coil.

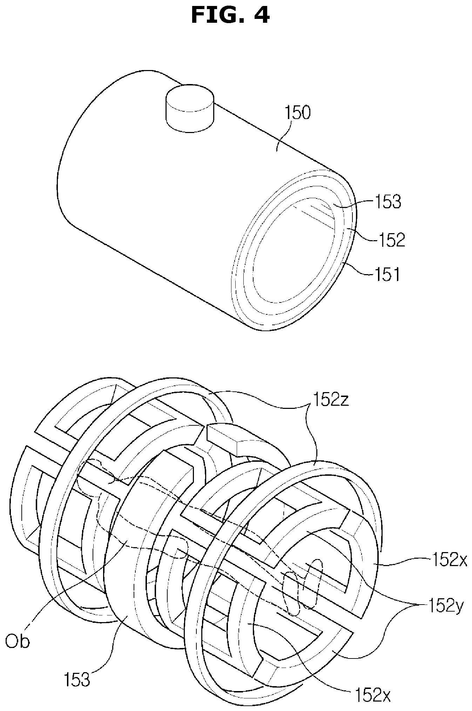

FIG. 2 illustrates an MRI scanner cutaway, FIG. 3 is a view illustrating a cross-section of an examination region, in which an object is placed, along the X, Y and Z axes, and FIG. 4 is view illustrating the structure of the magnet assembly and the gradient coil portion.

Referring to FIG. 2, the scanner (hereinafter referred to as magnet assembly 150) has a tube like structure and may be referred to as a gantry or a bore. The inner space of the magnet assembly 150 is referred to as a cavity, and the transfer table 200 transfers an object Ob positioned thereon to the cavity to acquire an MR image.

The magnet assembly 150 includes the static field coil portion 151, the gradient coil portion 152, and the RF transmission coil 153 as illustrated in FIGS. 1A or 1B.

The static field coil portion 151 may be formed in a shape in which a coil is wound around the cavity, and when current is applied to the static field coil portion 151, a static magnetic field is formed inside the magnet assembly 150, i.e., in the cavity.

The direction of the static magnetic field is substantially parallel with the driving axis of the magnet assembly 150.

When the static magnetic field is formed in the cavity, atomic nuclei of atoms constituting the object Ob, particularly, hydrogen atoms, are arranged in the direction of the static magnetic field and execute precession in the direction of the static magnetic field. The precession speed of atomic nuclei may be represented as a precession frequency, and such a precession frequency may be referred to as a Larmor frequency and expressed by Equation 1 below.

## [Equation 1]

$$\omega = \gamma B0$$

Here, $\omega$ is a Larmor frequency, $\gamma$ is a proportional constant which varies according to kinds of atomic nuclei, and B0 is the intensity of an external magnetic field measured in tesla (T) or gauss (G). The unit of the precession frequency is Hz.

For example, hydrogen protons have a precession frequency of 42.58 MHz in the external magnetic field of 1 T and, among elements constituting a human body, hydrogen occupies the largest percentage, and thus an MR signal is acquired predominantly using precession of hydrogen protons during MRI.

The gradient coil portion 152 generates gradients in the static magnetic field formed in the cavity, thus forming gradient magnetic fields.

As illustrated in FIG. 3, an axis running parallel with the lengthwise direction from the head to the feet of the object Ob, i.e., an axis running parallel with the direction of the static magnetic field, may be defined as the Z-axis, an axis running parallel with the lateral direction of the object Ob may be defined as the X-axis, and an axis running parallel with the vertical direction in the space may be defined as the Y-axis.

In order to acquire three dimensional (3D) spatial information of the MR signal, gradient magnetic fields in all directions of the X-axis, Y-axis and Z-axis are required. For example, the gradient coil portion 152 includes three pairs of gradient coils.

As illustrated in FIG. 4, Z-axis gradient coils 152z include a pair of ring type coils, and Y-axis gradient coils 152y are located above and below the object Ob. Further, X-axis gradient coils 152x are located at the left and right sides of the object Ob.

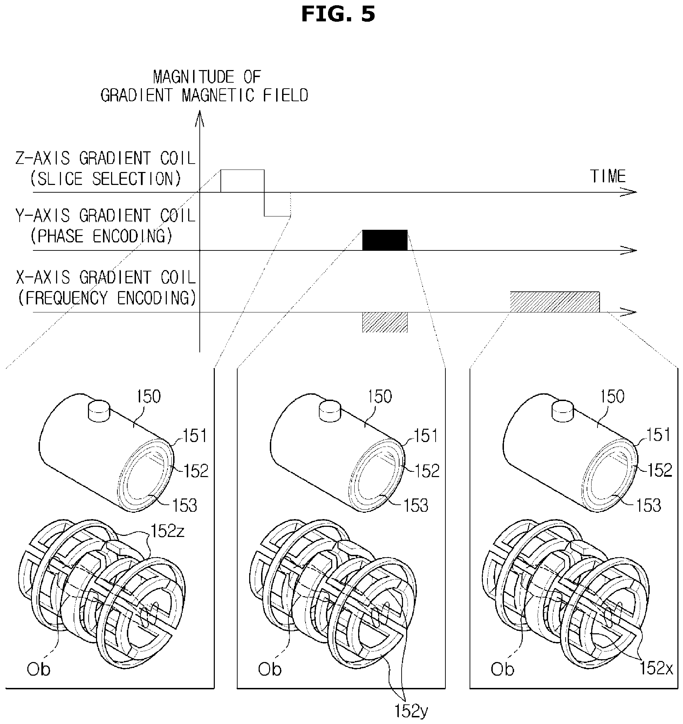

FIG. 5 is a view illustrating a pulse sequence regarding operations of the respective gradient coils of the gradient coil portion.

When direct currents having opposite polarities flow in the two Z-axis gradient coils 152z in opposite directions, the magnetic field is changed in the Z-axis direction and thus the gradient magnetic field is formed.

When the gradient magnetic field is formed by flowing current along the Z-axis gradient coils 152z for a predetermined time, the resonance frequency is changed to a higher frequency or a lower frequency according to the magnitude of the gradient magnetic field. When an RF signal corresponding to a specific position is applied through the RF transmission coil 153, only protons of a slice corresponding to the specific position resonate. Therefore, the Z-axis gradient coils 152z are used in slice selection. As the gradient magnetic field formed in the Z-axis direction is increased, a slice having a smaller thickness may be selected.

When the slice is selected through the gradient magnetic field formed by the Z-axis gradient coils 152z, all spins constituting the slice have the same frequency and the same phase and thus the respective spins are indistinguishable from one another.

In particular, when a gradient magnetic field in the Y-axis direction is formed by the Y-axis gradient coils 152y, the gradient field causes phase shift so that rows of the slice have different phases.

That is, when the Y-axis gradient magnetic field is formed, the phase of the spins of the row to which a large gradient magnetic field is applied is changed to a higher frequency, and the phase of the spins of the row to which a small gradient magnetic field is applied is changed to a lower frequency. When the Y-axis gradient magnetic field is removed, phase shift of the respective rows of the selected slice occurs and thus the rows have different phases, and thereby, the rows may be distinguished from one another. As described above, the gradient magnetic field formed by the Y-axis gradient coils 152y is used in phase encoding.

The slice is selected through the gradient magnetic field formed by the Z-axis gradient coils 152z, and the rows constituting the selected slice are distinguished from one another by different phases thereof through the gradient field formed by the Y-axis gradient coils 152y. However, the respective spins constituting each row have the same frequency and the same phase, and are thus indistinguishable from one another.

In particular, when a gradient magnetic field in the X-axis direction is formed by the X-axis gradient coils 152x, the X-axis gradient magnetic field causes the spins constituting each row to have different frequencies so that the respective spins are distinguishable from one another. As described above, the gradient magnetic field formed by the X-axis gradient coils 152x is used in frequency encoding.

As described above, the gradient magnetic fields formed by the Z-axis, Y-axis and X-axis gradient coils execute encoding of spatial positions of the respective spins, i.e., spatial encoding, through slice selection, phase encoding and frequency encoding.

The gradient coil portion 152 is connected to the gradient controller 130, and the gradient controller 130 applies a gradient waveform (i.e., a current pulse) to the gradient coil portion 152 according to a control signal transmitted from the pulse sequence controller 122 and then generates gradient magnetic fields. Therefore, the gradient controller 130 may be referred to as a gradient power supply, and may include three drive circuits corresponding to the three pairs of gradient coils 152x, 152y and 152z of the gradient coil portion 152.

Atomic nuclei arranged by the external magnetic field execute precession at the Larmor frequency, and the magnetization vector sum of several atomic nuclei may be represented as net magnetization M.

A Z-axis component of the net magnetization M may not be measured, and thus only Mxy may be detected. Therefore, in order to acquire an MR signal, the net magnetization may have to be present on the X-Y plane through excitation of the atomic nuclei. In order to excite the atomic nuclei, an RF pulse tuned to the Larmor frequency of the atomic nuclei may have to be applied to the static magnetic field.

The RF transmission coil 153 is connected to the RF transmitter 140, and the RF transmitter 140 applies a high frequency signal to the RF transmission coil 153 according to a control signal transmitted from the pulse sequence controller 122 so that the RF transmission coil 153 may transmit the RF pulse to the inside of the magnet assembly 150.

The RF transmitter 140 may include a modulation circuit modulating a high frequency signal into a pulse type signal, and an RF power amplifier amplifying the pulse type signal.

The RF reception coil 170 may receive an RF signal generated from atomic nuclei (i.e., an magnetic resonance (MR) signal). The RF reception coil 170 may transmit the MR signal to the image processor 16 through a switching portion and the image processor 16 may generate an MR image by processing the MR signal. Particularly, the image processor 16 includes the data receiver 161 collecting an MR signal from the RF reception coil and processing the MR signal, and the data processor generating an MR image using data received by the data receiver 161.

The data receiver 161 may include an amplifier amplifying the MR signal received by the RF reception coil 170.

As a method to acquire an MR signal from atomic nuclei, a spin echo pulse sequence is may be used. If the RF transmission coil 153 applies RF pulses, when an RF pulse is transmitted one more time at a proper time interval Δt after application of the first RF pulse, strong transverse magnetization of the atomic nuclei occurs after a time Δt therefrom has elapsed, and an MR signal may be acquired therefrom. This is referred to as a spin echo pulse sequence, and time taken to generate the MR signal after application of the first RF pulse is referred to as time echo (TE).

A flip degree of protons may be represented as an angle to which the protons move from the axis where the protons are located before flip, and be represented as a 90 degree RF pulse, a 180 degree RF pulse, etc. according to the flip degree of the protons.

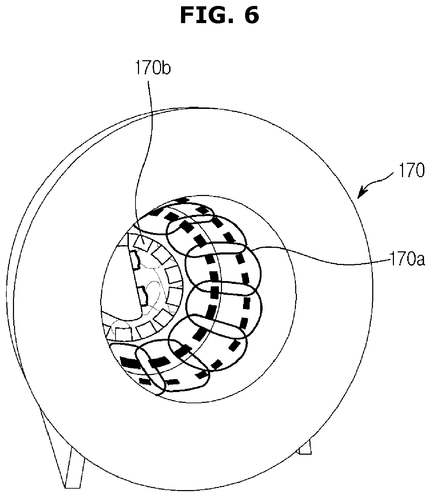

FIG. 6 is a view illustrating the RF reception coil and an overheating problem according to an exemplary embodiment.

In general, the RF reception coil 170 may be fixed to or removably fixed to the bore. Particularly, the removable RF reception coil 170 may vary according to the object (e.g., a target body part) to obtain an image. The RF reception coil 170 may include a head coil, a spine coil, a shoulder coil, a breast coil, a torso coil, a knee coil, a PV coil or a foot-ankle coil.

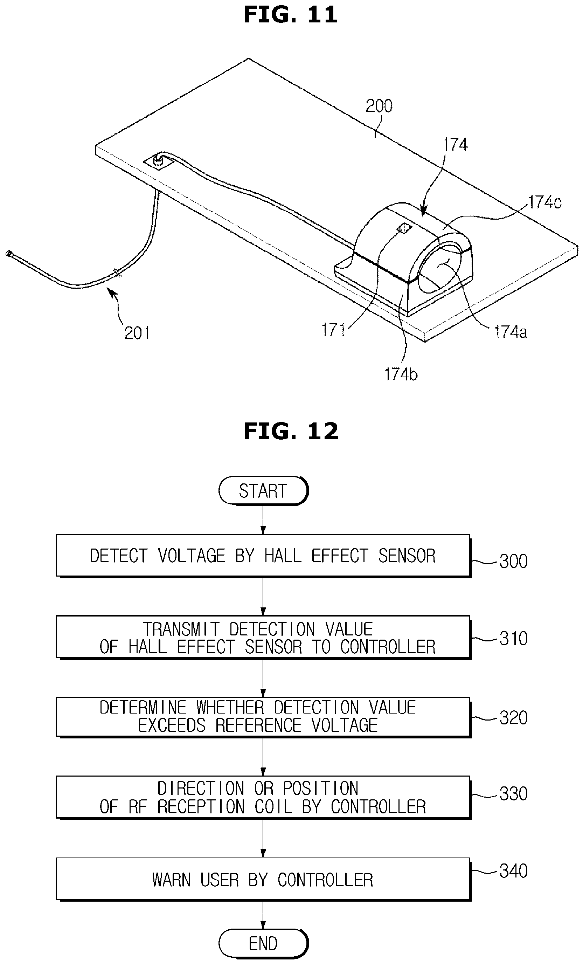

The RF reception coil 170 may be connected to the controller 120 by wire or wirelessly and may transmit the MR signal collected by the RF reception coil 170 to the controller 120 or an external device. An example in which the RF reception coil 170 transmits the MR signal will be described later with reference to FIG. 11.

The RF reception coil 170 may include RF coils of various channels such as 16 channels, 32 channels, 72 channels, and 144 channels. The channel represents a pathway capable of receiving an MR signal, and when the channel of the RF reception coil 170 is increased, the resonance signal acquisition time may be reduced and image data with a higher sensitivity can be generated.

According to an exemplary embodiment, the RF reception coil 170 may be produced as illustrated in FIG. 6. Each of circuit terminal illustrated in FIG. 6 schematically illustrates the arrangement of a coil 170a of the RF reception coil 170. In the RF reception coil 170 of FIG. 6, a circuit serving as an antenna to receive a MR signal and a local coil are arranged.

However, as the number of the channel is increased in the RF reception coil 170, the volume coil may be increased and it may cause the overheating when the power is applied.

As mentioned above, when the static field is formed in the cavity, the RF reception coil 170 receives an MR signal using the precession of the hydrogen proton in the object Ob. When the RF reception coil 170 is abnormally mounted to the object Ob or when the RF reception coil 170 is not mounted in a proper direction, it may be difficult to properly receive the MR signal. Therefore, this means that it may be difficult to obtain a clear image of the object Ob. In this case, a user may repeatedly operate the MRI apparatus 100 to obtain a clear image, and thus the excessive power may be applied to the RF reception coil 170.

Further, the RF reception coil 170 may be separately disposed from the magnet assembly 150 of the target object Ob to receive an MR signal efficiently. The separated RF receiving coil 170 is disposed in close contact with the object Ob.

When the RF reception coil 170 is abnormally attached to the object Ob, the RF reception coil 170 in close proximity to the object Ob may receive excessive power, and a circuit 170b embedded in the RF reception coil 170 may generate heat. Since the heated RF reception coil 170 is located closed to the object Ob, there is a risk of the accident.

FIG. 7 is a view illustrating a hall effect sensor according to an exemplary embodiment. Referring to FIG. 7, the disclosed RF reception coil 170 may include the hall effect sensor 171

Hall effect (hall-effect) means the potential difference symptoms. Particularly, when a magnetic field is formed perpendicular to the direction of the current in a state that a current flows through the conductor, a phenomenon that a potential difference is generated in the conductor through which the current flows (electric field is formed) is a hall effect.

An arrow that connects the N pole to the S-pole of the magnet forms a magnetic field as illustrated in FIG.

7. The N pole and the S pole mean the magnet assembly 150 of the MRI apparatus 100, and the plurality of arrows connecting the N pole and the S pole mean a static field.

When the hall effect sensor 171 receives the electric power from a power source 180, the current flows from a negative terminal to a positive terminal of the power source 180. That is, the current flows in the x-axis of FIG. 7. In this time, a potential difference is generated in the y-axis direction by the hall effect in a conductor 175 through which the current flows.

The hall effect sensor 171 detects the potential difference generated in the y-axis direction, i.e., a hall voltage.

A voltage value of the hall voltage detected by the hall effect sensor 171 may be changed by the magnetic field. Therefore, the hall effect sensor 171 may detect the intensity of the external magnetic field. The hall effect sensor 171 may apply the intensity of the external magnetic field to detect a direct current or an alternating current and use the intensity of the external magnetic field to detect the rotation of the rotor.

The detection value of the described hall effect sensor 171 provided in the RF reception coil 170 may be used to determine the position (e.g., the orientation) of the RF reception coil 170 in the static field. Particularly, the controller 120 of the MRI apparatus 100 (e.g., the RF coil position detector 124) may determine whether the RF reception coil 170 is placed in a proper direction in the static field, based on the potential value transmitted by the hall effect sensor 171.

According to an exemplary embodiment, the magnetic field sensor (e.g., the hall effect sensor 171) may be provided in the RF reception coil 170 together with a DC conductor. The DC conductor may serve as amplifying the potential value generated by the hall effect sensor. The DC conductor may amplify the detection value of the hall effect sensor 171 to help the RF coil position detector 124 so that the RF coil position detector 124 easily detects minute variations in the detection value.

In addition, the hall effect sensor 171 may be provided in the RF reception coil 170 with a different component, but is not limited thereto.

FIG. 8 is a view illustrating an RF reception coil according to an exemplary embodiment, and FIGS. 9A and 9B illustrate an operation of detecting the direction of a breast coil in the static field. To avoid redundant descriptions, a description thereof will be described together.

Referring to FIG. 8, the RF reception coil 170 may be implemented as a breast coil 172. The breast coil 172 having a body array may be placed in the chest of object Ob to receive an MR signal generated by internal organs such as the heart and organs of the human body.

According to an exemplary embodiment, the breast coil 172 may include the hall effect sensor 171 as well as a coil therein.

At least one hall effect sensor 171 a and 171 b may be provided in the breast coil 172, and the hole effect sensor 171 may be placed in any location of the breast coil 172 as long as placed inside the breast coil 172.

Referring to FIGS. 9A and 9B the breast coil 172 may be placed on the transfer table 200 and may move into the bore together with the transfer table 200.

The breast coil 172 may be positioned over the breast of the object Ob to image the breast, but for convenience of description, the object Ob is omitted in FIGS. 9A and 9B.

Hall effect sensors 171a and 171b embedded in the breast coil 172 measures a hall voltage value by the effect of the magnetic field inside the bore.

As mentioned with reference to FIG. 2, the static field may be formed inside the bore by the magnet assembly 150. The static field may be formed in an arrow direction (i.e., direction A), as illustrated in FIGS. 9A and 9B.

As illustrated in FIGS. 9A and 9B, when the breast coil 172 is moved in direction A, the hall effect sensors 171 a and 171b detect a hall voltage value generated by the hall effect, and transmit the hall voltage value to the RF coil position detector 124.

For convenience of description, it is described that the RF coil position detector 124 is separated from the controller 120, but the RF coil position detector 124 may be integrally formed with the controller 120 as a single processor. Therefore, the RF coil position detector 124 will be referred to as the controller 120.

The controller 120 may compare the transmitted voltage value with a predetermined reference value. When the transmitted voltage value is greater than or equal to the predetermined reference value, the controller 120 may determine that the breast coil 172 is properly attached to the object Ob and then may allow the breast coil 172 to continue to move in direction A.

When the breast coil 172 is positioned differently from a case illustrated in FIGS. 9A and 9B, (e.g., when the breast coil 172 is misplaced on the object Ob or when the breast coil 172 is attached to the object Ob in a direction opposite to A direction), the hall voltage value, which is detected by the hall effect sensors 171 a and 171 b due to the static field, may be different from a reference value.

That is, when the detection value transmitted by the hall effect sensors 171 a and 171b less than or is equal to the reference value, the controller 120 may determine that the breast coil 172 is not properly prepared, and then stop the operation of the transfer table 200 or warn it to the user or the operator of the magnet assembly 150.

According to another exemplary embodiment, the controller 120 may determine an increase rate of a magnetic field strength that is detected by the breast coil 172 while the transfer table 200 and the breast coil 172 are moving into the bore of the magnetic assembly 150, and may compare the determined increase rate to a predetermined increase rate of a magnetic field strength. If the increase rate that is detected by the breast coil 172 is less than the predetermined increase rate, the controller 120 may determine that the breast coil 172 is placed in an abnormal position.

A detail method of determining the position and orientation of the breast coil 172 based on the detection value of the hall effect sensors 171 a and 171 b will be described with reference to the drawings.

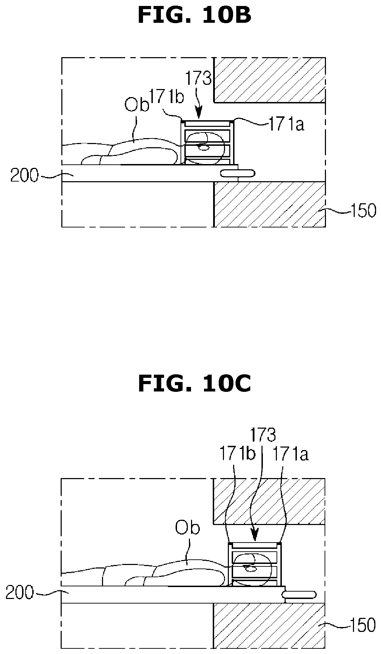

FIGS. 10A, 10B, and 10C illustrate a RF reception coil according to another exemplary embodiment.

Referring to FIG. 10A, the illustrated RF reception coil 170 is a head coil 173 for taking the brain images of an object Ob.

As illustrated in FIG. 10A, the head coil 173 may be provided in the head part of the object Ob and moved to the bore together with the transfer table 200. The head coil 173 may include a first hall effect sensor 171 a and a second hall effect sensor 171 b.

While the head coil 173 is moved to the bore, the plurality of hall effect sensors 171 a and 171b detects a hall voltage value and then transmits the detected plurality of hall voltage values to the controller 120.

Referring to FIG. 10B, the head coil 173 is moved into the bore by the transfer table 200. Particularly, the first hall effect sensor 171a is firstly moved to the inside of the bore and transfers the hall voltage value, which is increased by the static field, to the controller 120.

The controller 120 may determine that the head coil 173 enters the bore when the hall voltage value transmitted by the first hall effect sensor 171 a exceeds the reference value.

Referring to FIG. 10C, the transfer table 200 continues to move by a user's input. The second hall effect sensor 171 b of the head coil 173 also moves into the bore.

In this case, the second hall effect sensor 171b transmits the hall voltage value, which is increased by the static field, to the controller 120. The controller 120 determines whether the transmitted hall voltage value exceeds the reference value.

For example, the controller 120 may determine that the head coil 173 is abnormally put to the object Ob when the hall voltage value transmitted by the second hall effect sensor 171 b is different from the hall voltage value transmitted by the first hall effect sensor 171 a, or when the hall voltage value transmitted by the second hall effect sensor 171b does not exceed the reference value.

In addition, when the second hall effect sensor 171b firstly transmits a detection value after the transfer table 200 starts to move, the controller 120 may determine that the head coil 173 is put to the object Ob in an abnormal direction.

For another example, the controller 120 may determine the position of the RF reception coil based on a period of time in which the first and second hall effect sensors 171 a and 171b transmit the detection value, and the detection value variation. Particularly, while the transfer table 200 moves the object Ob into the bore, the controller 120 may receive the detection value transmitted by the first and second hall effect sensors 171 a and 171 b. Since the position of the first hall effect sensor 171 a, and the position of the second hall effect sensor 171b provided in the RF reception coil 170 that is moving, are different from each other, the controller 120 may determine the position of the RF reception coil 170 by comparing the detection value of each hall effect sensor 171 a and 171 b.

The controller 120 may determine that a difference between a first amplitude of a magnetic field (e.g., a value of a magnetic flux density) that is detected by the first hall effect sensor 171a and a second amplitude of a magnetic filed that is detected by the second hall effect sensor 171b, and may determine whether the difference between the first amplitude and the second amplitude is greater than a predetermined amplitude difference. If the difference between the first amplitude and the second amplitude is beyond a predetermined amplitude difference range, the controller 120 may determine that the RF reception coil 170 is placed in an abnormal position. The first hall effect sensor 171 a and the second hall effect sensor 171 b may output voltage values that respectively correspond to the first amplitude of the magnetic field and the second amplitude of the magnetic field.

Additionally, the controller 120 may determine a difference between a first direction of a magnetic field detected by the first hall effect sensor 171 a and a second direction of a magnetic filed that is detected by the second hall effect sensor 171b, and may determine whether the difference between the first direction and the second direction is within a predetermined range of a magnetic field direction difference. If the difference between the first direction and the second direction is outside the predetermined range, the controller 120 may determine that the RF reception coil 170 is placed in an abnormal position with respect to the object Ob.

Meanwhile, when determining that the head coil 173 is abnormally put to the object Ob or when determining that the position of the head coil 173 is different in the bore, the controller 120 may stop the operation of the transfer table 200 or warn the user by using an alarm or the display.

FIG. 11 is a view illustrating the RF reception coil and the transfer table in accordance with an exemplary embodiment.

Referring to FIG. 11, the MRI apparatus 100 may include the transfer table 200 and the RF reception coil 170 for taking the knee image of the object Ob, i.e., a knee coil 174.

The knee coil 174 of FIG. 11 is a type of the RF reception coil 170 receiving an MR signal. The knee coil 174 may be formed in a circular shape to image the knee, and provided with a hollow 174a to which the knee is inserted.

The knee coil 174 may include a fixation portion 174b fixed to the bottom of the transfer table 200, and a casing 174c removably mounted to the fixation portion 174b and configured to form the hollow 174a by being coupled to the fixation portion 174b.

When the leg of the object Ob is placed in the fixation portion 174b, the casing 174c is coupled to the fixation portion 174b so that the leg of the object Ob is fixed not to move the leg.

However, although the knee coil 174 is fixed to the object Ob, the position of the object Ob may be misplaced due to the movement of the object Ob. In order to prevent such a case, the disclosed knee coil 174 includes the hall effect sensor 171. That is, the MRI apparatus 100 may determine whether the knee coil 174 is properly fixed or not or whether the direction of the casing 174c of the knee coil 174 is properly coupled or not, based on the detection value transmitted by the hall effect sensor 171.

The hall effect sensor 171 may be provided in the casing 174c of the knee coil 174 or coupled to the outside of the casing 174c of the knee coil 174, but is not limited thereto.

FIG. 11 illustrates that the transfer table 200 moves the object Ob to the cavity.

The transfer table 200 may be moved by the input of the user, or controlled by the transfer controller 123. For an example, when the RF coil position detector 124 determines that the knee coil 174 is not fixed based on the detection value of the hall effect sensor 171, the transfer controller 123 may stop the movement of the transfer table 200.

The transfer controller 123 may control the transfer table 200 through a connection portion 201 as illustrated in FIG. 11. Further, the connection portion 201 may deliver the power to the knee coil 174 and transmit an MR signal received by the knee coil 174 to the data receiver 161.

The transfer table 200 and the knee coil 174 illustrated in FIG. 11 are merely an example of the present disclosure and thus various modifications may be allowed as long as the knee coil 174 includes the hall effect sensor 171.

FIG. 12 is a flow chart illustrating an operation of the MRI apparatus.

First, the hall effect sensor 171 of the RF reception coil 170 detects the hall voltages in the bore (operation 300).

As mentioned above, the hall effect sensor 171 detects the hall voltage caused by the static field of the bore generated by the static field coil portion 151. The hall effect sensor 171 transmits the detected hall voltage value to the controller 120, particularly, the RF coil position detector 124 (operation 310).

The controller 120 determines whether the transmitted hall voltage value is the equal to or greater than the reference voltage (operation 320).

Particularly, the controller 120 may determine that the RF reception coil 170 is properly attached to the object Ob when the hall voltage value detected by the hall effect sensor 171 exceeds a threshold voltage.

Meanwhile, the reference voltage may be a predetermined value, and the reference voltage may vary according to the size of the static field emitted from the MRI apparatus 100.

The controller 120 may determine the direction or orientation of the RF reception coil 170 (operation 330).

For an example, when a single hall effect sensor 170 is provided in the neck part of the spine coil, the controller 120 may determine whether the object Ob properly wears the spine coil, based on a point of time in which the transfer table 200 is started and a moving time of the transfer table 200. For example, when the hall effect sensor 171 transmits a hall voltage value exceeding the threshold voltage after a reference time is expired, the controller 120 may determine that the object Ob inversely wears the spine coil.

For another example, the RF reception coil 170 may include at least one hall effect sensor 171. When the RF reception coil 170 is the spine coil for imaging the spine part of the object Ob, the spine coil may be manufactured with the orientation to distinguish the spine, i.e., from the neck to the waist.

The controller 120 may determine the direction of the RF reception coil 170 by comparing each detection value transmitted by the plurality of hall effect sensors 171 with the predetermined reference value.

In addition, the controller 120 may determine the position of the RF reception coil 170 by comparing each detection value transmitted by the plurality of hall effect sensors 171 with each other. When the RF reception coil 170 is moved into the bore, each hall effect sensor 171 may transmit different detection values according to the position thereof. The controller 120 may determine the position of the RF reception coil 170 by comparing the detection value with each other.

When it is determined that the RF reception coil 170 is not properly mounted, the controller 120 may warn the user (operation 340).

Alternative to operations 320 and 330 or in addition to operations 320 and 330, the controller 120 may determine an increase rate of a magnetic field strength that is detected by the RF reception coil 170 while the transfer table 200 and the RF reception coil 170 are moving into the bore of the magnetic assembly 150, and may compare the determined increase rate to a predetermined increase rate of a magnetic field strength. If the determined increase rate is less than the predetermined increase rate, the controller 120 may determine that the RF reception coil 170 is placed in an abnormal position, and may warn the user through an image or sound (operation 340).

For example, the controller 120 may display a warning message to the user on the display 112 or output a warning sound through the sound output portion 113. The controller 120 may stop the object Ob into the bore to control the transfer table 200. The controller 120 may stop the object Ob from entering to the bore by controlling the transfer table 200.

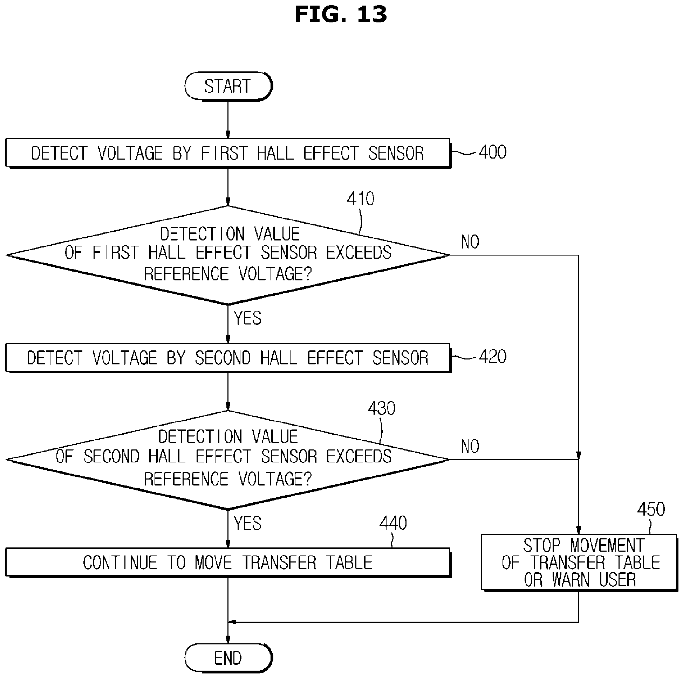

FIG. 13 is a flowchart illustrating a method for determining the orientation of the RF reception coil.

According to an exemplary embodiment, the RF reception coil 170 may include the first hall effect sensor 171 a and the second hall effect sensor 171 b, as illustrated in FIG. 8.

As mentioned above, the RF reception coil 170 may be moved to the bore while being attached to the object Ob. The first hall effect sensor 171a detects the hall voltage due to the static field (operation 400).

The controller 120 receives a first detection value detected by the first hall effect sensor 171a and determines whether the first detection value exceeds the reference voltage (operation 410).

When the first detection value does not exceed the reference voltage, the controller 120 may determine that the RF reception coil 170 is not properly attached to the object Ob. The controller 120 may stop the movement of the transfer table 200 and warn the user (operation 450).

When the first detection value exceeds the reference voltage, the controller 120 may continue to move the transfer table 200 into the bore.

The second hall effect sensor 171b detects the hall voltage value due to the strong static field in the bore (operation 420).

The second detection value detected by the second hall effect sensor 171b is transmitted to the controller 120, and the controller 120 compares the second detection value with the predetermined reference voltage value.

The controller 120 determines whether the second detection value exceeds the reference voltage (operation 430).

When the second detection value exceeds the reference voltage, the controller 120 may determine that the RF reception coil 170 is attached to the object Ob in a normal direction or in a normal position.

In this case, the controller 120 may control the transfer table 200 so that the transfer table 200 continues to move into the bore until the transfer table 200 is completely placed in the bore (operation 440).

When the first detection value does not exceed the reference voltage, the controller 120 may determine that the RF reception coil 170 is attached to the object Ob in an abnormal direction or in an abnormal position. In this case, the controller 120 may stop the movement of the transfer table 200 and warn it to the user (operation 450).

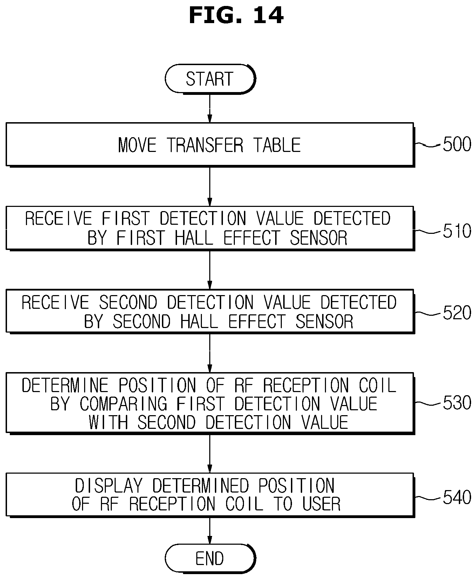

FIG. 14 is a flowchart illustrating a method for determining the position of the RF reception coil.

Referring to FIG. 14, the controller 120 moves the transfer table 200 (500).

With the transfer table 200, the RF reception coil 170 attached to the object Ob may be moved to the bore. While the transfer table 200 moves, the RF reception coil 170 also moves and the hall effect sensor 171 a and 17b provided in the RF reception coil 170 detects the hall voltage due to the static field.

The controller 120 receives a first detection value detected by the first hall effect sensor 171 a (operation 510).

The controller 120 receives a second detection value detected by the second hall effect sensor 171b (operation 520).

The static field applied by the magnet assembly 150 cannot always uniform inside the bore. In addition, the hall voltage value, which is detected by the plurality of hall effect sensors 171 a and 171 b, which are apart from each other in the RF reception coil 170, may vary according to the position of the RF reception coil 170 moving to the inside of the bore.

That is, the controller 120 determines the position of the RF reception coil 170 by comparing the first detection value and the second detection value (operation 530).

As illustrated in FIGS. 10A, 10B, and 10C, the first detection value of the first hall effect sensor 171 a that is strongly affected by the static field in the RF reception coil 170 may be different from the detection value detected by the second hall effect sensor 171b. The controller 120 may determine the position of the RF reception coil 170 by comparing the detection values.

The controller 120 displays the determined position of the RF reception coil 170 to the user (operation 540).

A method in which the controller 120 transmits the position information of the RF reception coil 170 to a user may vary. For example, the controller 120 may display the position of the RF reception coil 170 by outputting an image or figures on the display 112.

According to the controlling method, the MRI apparatus 100 determines the direction and position of the RF reception coil 170, and the MRI apparatus 100 may inform the user whether the RF reception coil 170 is properly mounted to the object Ob. Accordingly, the MRI apparatus 100 may prevent a case in which a clear image is not output since the RF reception coil 170 is incorrectly mounted, and the MRI apparatus 100 may prevent an accident of the object Ob by minimizing the heat which is generated in the coil of the RF reception coil 170.

According to the above-described exemplary embodiments, the magnetic resonance imaging apparatus and a controlling method thereof may allow to determine the position or the orientation of the RF coil in the bore, by using the magnetic field sensor contained in the RF coil, and thus it may be possible to prevent the re-imaging caused by the erroneous mounting and to secure the patient safety by preventing the overheat of the RF coil.

While not restricted thereto, an exemplary embodiment can be embodied as computer-readable code on a computer-readable recording medium. The computer-readable recording medium is any data storage device that can store data that can be thereafter read by a computer system. Examples of the computer-readable recording medium include read-only memory (ROM), random-access memory (RAM), CD-ROMs, magnetic tapes, floppy disks, and optical data storage devices. The computer-readable recording medium can also be distributed over network-coupled computer systems so that the computer-readable code is stored and executed in a distributed fashion. Also, an exemplary embodiment may be written as a computer program transmitted over a computer-readable transmission medium, such as a carrier wave, and received and implemented in general-use or special-purpose digital computers that execute the programs. Moreover, it is understood that in exemplary embodiments, one or more units of the above-described apparatuses and devices can include circuitry, a processor, a microprocessor, etc., and may execute a computer program stored in a computer-readable medium.

The foregoing exemplary embodiments are merely exemplary and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

**Claims**

1. A magnetic resonance imaging (MRI) apparatus comprising:

    a magnet assembly configured to generate a magnetic field in an inner space of the magnet assembly;
    a radiofrequency (RF) reception coil configured to receive a magnetic resonance (MR) signal excited from an object to which the magnetic field is applied;
    a magnetic sensor disposed at the RF reception coil and configured to detect a voltage value indicating a static field generated by the magnet assembly; and
    a processor configured to determine whether the RF reception coil is placed in a normal position based on the voltage value transmitted from the magnetic sensor when the RF reception coil enters the inner space of the magnetic assembly.

2. The MRI apparatus of claim 1, wherein the processor is further configured to determine that the RF reception coil is placed in the normal position in response to the voltage value transmitted from the magnetic sensor exceeding a predetermined reference voltage value.

3. The MRI apparatus of claim 1, wherein the RF reception coil is separated from the magnet assembly and disposed closer to the object than the magnet assembly.

4. The MRI apparatus of claim 1, wherein the RF reception coil comprises a direct current (DC) conductor that is coupled to the magnetic sensor.

5. The MRI apparatus of claim 1, further comprising:

    a transfer table configured to transfer the object to the inner space of the magnet assembly.

6. The MRI apparatus of claim 5, wherein the processor is further configured to stop the transfer table from moving in response to determining that the RF reception coil is placed in an abnormal position.

7. The MRI apparatus of claim 1, further comprising:

    an image processor configured to generate an MR image based on MR signals transmitted from the RF reception coil; and
    a display configured to display the MR image generated by the image processor,
    wherein the processor is further configured to control the display to display a warning image in response to determining that the RF reception coil is placed in an abnormal position.

8. The MRI apparatus of claim 1, further comprising:

    a sound output interface configured to output a warning sound to a user in response to determining that the RF reception coil is placed in an abnormal position.

9. The MRI apparatus of claim 1, wherein the magnetic sensor comprises a first magnetic sensor and a second magnetic sensor that are disposed at the RF reception coil, and the first magnetic sensor is disposed apart from the second magnetic sensor.

10. The MRI apparatus of claim 9, wherein the processor is further configured to determine whether the RF reception is placed in the normal position by comparing a first detection value of the first magnetic sensor and a second detection value of the second magnetic sensor with a predetermined reference voltage value.

11. The MRI apparatus of claim 9, wherein the processor is further configured to determine whether the RF reception is placed in the normal position by comparing a first detection value of the first magnetic sensor with a second detection value of the second magnetic sensor.

**12.** A control method of a magnetic resonance imaging (MRI) apparatus, the control method comprising:

generating, by a magnet assembly, a magnetic field in an inner space of the magnetic assembly;

receiving, by a radiofrequency (RF) reception coil, a magnetic resonance (MR) signal excited from an object to which the magnetic field is applied;

detecting, by a magnetic sensor, a voltage value indicating a static field generated by the magnetic assembly; and

determining whether the RF reception coil is placed in a normal position based on the voltage value transmitted from the magnetic sensor when the RF reception coil enters the inner space of the magnetic assembly.

**13.** The control method of claim 12, wherein the determining comprises determining that the RF reception coil is placed in the normal position in response to the voltage value transmitted from the magnetic sensor exceeding a predetermined reference voltage value.

**14.** The control method of claim 12, further comprising:

stopping the object from moving into the inner space of the magnet assembly in response to determining that the RF reception coil is placed in an abnormal position.

**15.** The control method of claim 12, wherein the magnetic sensor includes a first magnetic sensor and a second magnetic sensor that are disposed at the RF reception coil, and the first magnetic sensor is disposed apart from the second magnetic sensor, and

the determining comprises determining whether the RF reception is placed in the normal position by comparing a first detection value of the first magnetic sensor and a second detection value of the second magnetic sensor with a predetermined reference voltage value.

**FIG. 1A**

# FIG. 1B

# FIG. 2

# FIG. 3

# FIG. 4

# FIG. 5

# FIG. 6

# FIG. 7

# FIG. 8

# FIG. 9A

# FIG. 9B

# FIG. 10A

## FIG. 10B

## FIG. 10C

# FIG. 11

# FIG. 12

```
                    ( START )
                        │
                        ▼
┌──────────────────────────────────────────────┐
│   DETECT VOLTAGE BY HALL EFFECT SENSOR         │──300
└──────────────────────────────────────────────┘
                        │
                        ▼
┌──────────────────────────────────────────────┐
│         TRANSMIT DETECTION VALUE               │──310
│   OF HALL EFFECT SENSOR TO CONTROLLER          │
└──────────────────────────────────────────────┘
                        │
                        ▼
┌──────────────────────────────────────────────┐
│     DETERMINE WHETHER DETECTION VALUE          │──320
│         EXCEEDS REFERENCE VOLTAGE              │
└──────────────────────────────────────────────┘
                        │
                        ▼
┌──────────────────────────────────────────────┐
│           DIRECTION OR POSITION                │──330
│   OF RF RECEPTION COIL BY CONTROLLER           │
└──────────────────────────────────────────────┘
                        │
                        ▼
┌──────────────────────────────────────────────┐
│        WARN USER BY CONTROLLER                 │──340
└──────────────────────────────────────────────┘
                        │
                        ▼
                    ( END )
```

# FIG. 13

START

DETECT VOLTAGE BY FIRST HALL EFFECT SENSOR ~400

DETECTION VALUE OF FIRST HALL EFFECT SENSOR EXCEEDS REFERENCE VOLTAGE? ~410 — NO

YES

DETECT VOLTAGE BY SECOND HALL EFFECT SENSOR ~420

DETECTION VALUE OF SECOND HALL EFFECT SENSOR EXCEEDS REFERENCE VOLTAGE? ~430 — NO

YES

CONTINUE TO MOVE TRANSFER TABLE 440

STOP MOVEMENT OF TRANSFER TABLE OR WARN USER 450

END

# FIG. 14

```
            ┌─────────┐
            │  START  │
            └────┬────┘
                 │
                 ▼
┌──────────────────────────────────────────┐
│          MOVE TRANSFER TABLE             │───500
└──────────────────────┬───────────────────┘
                       │
                       ▼
┌──────────────────────────────────────────┐
│  RECEIVE FIRST DETECTION VALUE DETECTED   │───510
│       BY FIRST HALL EFFECT SENSOR         │
└──────────────────────┬───────────────────┘
                       │
                       ▼
┌──────────────────────────────────────────┐
│  RECEIVE SECOND DETECTION VALUE DETECTED  │───520
│      BY SECOND HALL EFFECT SENSOR         │
└──────────────────────┬───────────────────┘
                       │
                       ▼
┌──────────────────────────────────────────┐
│  DETERMINE POSITION OF RF RECEPTION COIL  │───530
│     BY COMPARING FIRST DETECTION VALUE    │
│        WITH SECOND DETECTION VALUE        │
└──────────────────────┬───────────────────┘
                       │
                       ▼
┌──────────────────────────────────────────┐
│       DISPLAY DETERMINED POSITION         │───540
│     OF RF RECEPTION COIL TO USER          │
└──────────────────────┬───────────────────┘
                       │
                       ▼
                 ┌─────────┐
                 │   END   │
                 └─────────┘
```

Europäisches
Patentamt

European
Patent Office

Office européen
des brevets

# EUROPEAN SEARCH REPORT

Application Number

EP 17 20 4473

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/176809 A1 (BIBER STEPHAN [DE] ET AL) 15 July 2010 (2010-07-15) | 1,3-5,7, 8,12 | INV. G01R33/28 |
| A | * paragraphs [0024] - [0033], [0039], [0040]; claims 1-8 * | 6,11,14 | |
| X | US 2013/119981 A1 (CHOI SEUNG JE [KR] ET AL) 16 May 2013 (2013-05-16) | 1-5,7-9, 12,13,15 | |
| A | * paragraph [0093] - paragraph [0096]; claims 1-5 * | 6,11,14 | |
| X | US 2016/178713 A1 (FISCHER HUBERTUS [DE] ET AL) 23 June 2016 (2016-06-23) | 1,3-5,9, 10,12,15 | |
| A | * paragraphs [0007] - [0009], [0013] - [0016] * | 6,11,14 | |

TECHNICAL FIELDS
SEARCHED        (IPC)

G01R

The present search report has been drawn up for all claims

| Place of search | Date of completion of the search | Examiner |
|---|---|---|
| Munich | 7 June 2018 | Skalla, Jörg |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 20 4473

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2018

| Patent document cited in search report | | Publication date | Patent family member(s) | | Publication date |
|---|---|---|---|---|---|
| US 2010176809 | A1 | 15-07-2010 | DE 102009004183 A1<br>US 2010176809 A1 | | 22-07-2010<br>15-07-2010 |
| US 2013119981 | A1 | 16-05-2013 | CN 103105596 A<br>KR 20130051685 A<br>US 2013119981 A1 | | 15-05-2013<br>21-05-2013<br>16-05-2013 |
| US 2016178713 | A1 | 23-06-2016 | CN 105717468 A<br>DE 102014226761 A1<br>US 2016178713 A1 | | 29-06-2016<br>23-06-2016<br>23-06-2016 |

For more details about this annex : see Official Journal of the European Patent Office, No. 12/82